# FASCICULE DE BREVET EUROPEEN

(11) **EP 0 814 818 B1**
(45) Date de publication et mention de la délivrance du brevet: **09.02.2000**
(21) Numéro de dépôt: 96906817.0
(22) Date de dépôt: 12.03.1996
(51) Int. Cl.: A61K 33/30, A61K 33/34, A61K 33/14

(54) **COMPOSITIONS PHARMACEUTIQUES POUR LA REGULATION DE LA POLARISATION DES MUSCLES LISSES UNITAIRES**
PHARMAZEUTISCHE ZUSAMMENSETZUNGEN ZUR POLARISATIONSREGULIERUNG DER EINHEITLICHEN GLATTEN MUSKELN
PHARMACEUTICAL COMPOSITIONS FOR CONTROLLING UNITARY SMOOTH MUSCLE POLARISATION

(30) Priorité: 13.03.1995 FR 9502880
(43) Date de publication de la demande: 07.01.1998
(73) Titulaire: Drouault, Guy, 91570 Bièvres (FR)
(72) Inventeur: Drouault, Guy, 91570 Bièvres (FR)
(74) Mandataire: Bonneau, Gérard
(86) Numéro de dépôt international: FR9600382
(87) Numéro de publication internationale: WO9628172

(56) Documents cités:
- WO-A-84/00298
- FR-A- 2 602 678

## Description

La présente invention a pour objet des compositions pharmaceutiques pour la régulation de la polarisation des muscles lisses unitaires. L'invention concerne aussi une trousse contenant au moins deux des compositions pharmaceutiques ci-dessus.

Dans le brevet FR 2 602 678 on a déjà décrit des compositions pharmaceutiques aqueuses à base de sels de magnésium, sodium et potassium pour la régulation des circulations locales, qui conviennent notamment dans le traitement des troubles et affections récidivants des voies respiratoires supérieures.

Ces compositions sont constituées d'une solution aqueuse contenant des sels de magnésium, de potassium, de sodium et éventuellement de calcium en combinaison avec au moins un sucre et au moins un acide aminé.

Le sucre contenu dans ces compositions est un sucre absorbable choisi parmi le glucose, le galactose et le xylose ou éventuellement un sucre non absorbable, tel que le mannitol.

L'acide aminé contenu dans ces compositions est choisi parmi l'arginine, la phénylalanine et l'acide glutamique.

Les voies respiratoires, outre leur rôle de conduction de l'air inspiré, ont la charge de traduire et de traiter les informations aéroportées venues du monde extérieur. Leur mission essentielle est de débarrasser l'air inspiré des particules inertes ou vivantes qu'il contient. Pour cela, elles déploient à leur surface plusieurs barrières destinées à neutraliser les contaminants aéroportés :
- la barrière muco-ciliaire
- la barrière des germes commensaux
- la barrière des IgA sécrétoires.

Mais, alors que tout au long des voies respiratoires ce piégeage est laissé au hasard d'un contact des contaminants avec la paroi, à l'étage rhinosinusien, au contraire, il existe une structure dynamique, constituée d'un réseau vasculaire très dense avec du tissu érectile, et dont les variations de volume ont un double effet : canaliser et précipiter les contaminants aériens sur la barrière mobile muco-ciliaire, et brancher les surfaces sinusiennes sur le circuit respiratoire.

L'animation de cet appareil est assurée par du tissu musculaire lisse unitaire. Les muscles lisses unitaires ont la particularité de se contracter spontanément, excités seulement par des facteurs locaux (facteurs physiques : l'étirement par exemple, facteurs chimiques ou métaboliques). Ils animent la microcirculation et la fonction mucociliaire des muqueuses respiratoires. Quelle que soit la nature de l'information reçue, elle modifie la polarité membranaire de ce tissu musculaire et génère des potentiels d'action auxquels est associée une contraction. Cette activité automatique est initiée dans des cellules stimulatrices (de type "pacemaker") localisées de façon variable dans le tissu et se propage à tout le tissu comme s'il s'agissait d'une structure unique.

Cette activité spontanée seulement modulée mais non déclenchée par leur innervation, permet une autorégulation locale. C'est de ce tissu que sont constituées les cellules musculaires des sphincters capillaires et de l'anastomose artério-veineuse, les myofibrilles des cellules et glandes sécrétoires et les protéines contractiles des cellules ciliées. C'est ainsi que cet appareil peut être autorégulé par le courant aérien et les contaminants aéroportés qui apportent l'information physique, et par des facteurs métaboliques locaux qui apportent l'information pharmacologique, le système nerveux autonome n'intervenant que pour des actions ponctuelles de défense ou d'adaptation de la muqueuse.

La transduction de ces informations n'est pas biochimique mais biophysique : il y a génération spontanée de potentiels d'action quelles que soient les informations : physiques, chimiques ou métaboliques. A la salve des potentiels d'action est associée une contraction du tissu musculaire lisse unitaire animant la microcirculation et l'ensemble muco-ciliaire ; et cette contraction est non seulement bien synchronisée mais également modulée par l'excitation et adaptée à l'excitation grâce aux potentiels d'action qui peuvent varier en nombre, amplitude et fréquence. Cette contraction dite phasique est la contraction fonctionnelle.

Ce sont des variations excessives de la polarité membranaire qui vont dysréguler cet appareil. De fortes excitations ou de fortes inhibitions apportées par des facteurs endogènes et (ou) des facteurs exogènes peuvent faire franchir à la membrane musculaire un seuil de polarisation ou de dépolarisation au-delà duquel la génération spontanée des potentiels d'action est abolie. Alors s'installe une contraction tonique, uniforme et dysrégulatrice, car elle dépend de mouvements ioniques calciques passifs non modulés par l'information.

Selon les facteurs étiologiques, l'abolition des potentiels d'action peut dans un premier temps dysréguler électivement la fonction microcirculatoire ou la fonction mucociliaire et engendrer ainsi divers processus pathogènes. Deux sont particulièrement importants par leur fréquence et leur rôle majeur dans l'initiation des troubles et affections des voies respiratoires supérieures.

La dysrégulation de la contraction d'un ou des deux sphincters capillaires ou de la tunique musculaire des anastomoses artério-veineuses entraîne un trouble vasomoteur d'expression clinique variable : dans le premier cas, par exemple, c'est d'abord un oedème mécanique, mais les perturbations ioniques et métaboliques déclenchées sont génératrices d'oedème tissulaire.

La dysrégulation de la contraction des protéines contractiles muco-ciliaires entraîne une hyper- ou une hypo-sécrétion avec dyskinésie ciliaire. La dessiccation muqueuse, en particulier, est un trouble redoutable. En l'absence de barrière mucociliaire, les contaminants vont se comporter comme des particules aéroportées et vont, au contact de la muqueuse - soit déclencher une réaction immunitaire, s'ils s'y attachent ou la pénètrent - soit manifester une action physique pathogène. Cette action varie avec la charge, la masse, la vitesse et l'angle d'impact des contaminants. Que l'action manifestée soit mécanique, calorique ou électromagnétique, elle est source de perturbations hémodynamiques et de réactions inflammatoires non spécifiques, avec troubles vasomoteurs.

Les compositions pharmaceutiques selon l'invention permettent de réguler la polarisation des muscles lisses unitaires dérégulés par une hyperpolarisation ou par une hyperdépolarisation ; elles provoquent ainsi la régénération spontanée de potentiels d'action et la restauration de la contraction phasique desdits muscles lisses unitaires, décapitant ainsi les mécanismes responsables de l'initiation du trouble vasomoteur, du trouble sécrétoire et par voie de conséquence, de l'inflammation non spécifique.

Les compositions pharmaceutiques selon l'invention sont des solutions aqueuses contenant des sels de magnésium, de potassium, de sodium et éventuellement de calcium, au moins un sucre et au moins un acide aminé essentiel en combinaison avec un sel de cuivre ou un sel de zinc.

Les sels de magnésium, de potassium, de sodium et éventuellement de calcium mis en oeuvre dans la composition selon l'invention sont des sels pharmaceutiquement acceptables. Ils peuvent être par exemple des chlorures, des sulfates, des phosphates ou des lactates.

Les sucres sont mis en oeuvre sous la forme dextrogyre.

A titre d'exemples de sucres appropriés aux fins de l'invention, on peut citer tous les sucres absorbables tels que le glucose, le galactose et le xylose et leurs analogues structuraux ayant un hydroxyle en position a sur le carbone 2.

On peut également utiliser en combinaison avec un sucre absorbable un sucre non absorbable, tel que le mannitol, qui favorise le développement d'un gradient électro-chimique. C'est aussi un bon capteur de radicaux libres OH^{•}.

Les acides aminés qui entrent dans la composition de l'invention sont des composés glucogéniques. On utilisera de préférence la forme lévogyre.

A titre d'exemples d'acides aminés appropriés aux fins de l'invention, on peut citer la L-phénylalanine, la L-arginine et l'acide glutamique. On notera que la L-phénylalanine est à la fois glucogénique et cétogénique. La quantité d'acide aminé peut varier entre 1 et 20 g par litre.

La quantité de sels à mettre en oeuvre dans la composition selon l'invention peut varier dans de larges limites. Toutefois, on indiquera qu'avantageusement la concentration en sels peut aller d'environ 10 mg par litre jusqu'à 2000 mg par litre et est de préférence comprise entre 40 et 800 mg par litre. La concentration en sel de potassium lorsque l'on utilise le chlorure de potassium est avantageusement comprise entre 10 mg par litre et 300 mg par litre.

La quantité totale de sucre dans la composition selon l'invention peut varier entre 200 et 10 000 mg par litre. Lorsqu'on utilise un sucre non absorbable, sa concentration est de l'ordre de 200 à 900 mg par litre.

Comme indiqué précédemment, les sels de magnésium, sodium et potassium sont essentiels aux fins de l'invention, alors que les sels de calcium ne sont pas indispensables.

On notera que selon le rapport pondéral K/Mg on obtient des compositions ayant un effet dépolarisant ou un effet polarisant sur les muscles lisses unitaires. Ainsi, si le rapport K/Mg est inférieur à 1, de préférence compris entre 0,40 et 0,85, la composition selon l'invention aura un effet dépolarisant alors qu'avec un rapport pondéral K/Mg compris entre 14 et 16 on obtiendra une composition ayant un effet polarisant. Dans ce dernier cas, on utilise de préférence comme sels des chlorures, en choisissant avantageusement le rapport pondéral K/Na entre 30 et 40.

Les sels de cuivre et de zinc appropriés aux fins de l'invention sont choisis parmi les sels hydrosolubles de ces métaux comme par exemple, le sulfate, le gluconate, le digluconate, le carbonate, etc, le gluconate de cuivre et le chlorure de zinc étant particulièrement préférés.

Les quantités de zinc métal et de cuivre métal peuvent varier entre 5 et 120 mg/l pour le zinc et 5 et 100 mg/l pour le cuivre.

Les compositions pharmaceutiques selon l'invention sont particulièrement indiquées dans le traitement des troubles vasomoteurs, des troubles sécrétoires et des troubles inflammatoires aigus et surtout subaigus des muqueuses respiratoires supérieures car elles régulent la polarisation des muscles lisses unitaires animant la microcirculation et la fonction mucociliaire de ces muqueuses, et rétablissent le mécanisme biophysique physiologique de transduction de l'information au sein de ces tissus.

En particulier, elles permettent de restaurer la perméabilité des fosses nasales, des sinus et des trompes d'Eustache, même en cas de surinfection virale ou de surinfection bactérienne.

Les compositions pharmaceutiques selon l'invention sont également indiquées dans le traitement des affections trachéo-bronchiques et de l'asthme intrinsèque (ou non allergique). En effet, la dérégulation des muscles lisses unitaires est responsable des troubles vasomoteurs, des troubles sécrétoires et de l'inflammation non spécifique, qui vont modifier la lumière trachéo-bronchique.

Les compositions pharmaceutiques selon l'invention permettent également de perméabiliser les canaux lacrymaux.

Selon un autre aspect, l'invention concerne également une trousse pour la régulation de la polarisation des muscles lisses unitaires, cette trousse comprenant au moins deux compositions pharmaceutiques telles que définies ci-dessus. Les compositions pharmaceutiques contenues dans cette trousse peuvent être administrées simultanément, successivement ou séparément, de préférence successivement. De préférence, la trousse selon l'invention comprend deux compositions pharmaceutiques selon l'invention, une ayant un effet polarisant sur les muscles lisses unitaires et l'autre ayant un effet dépolarisant sur les muscles lisses unitaires.

Les compositions selon l'invention peuvent être conditionnées sous différentes formes, telles que par exemple sous la forme atomisée pour l'utilisation en spray nasal ou en spray bronchique ou sous la forme d'ampoule pour bains nasals ou lavages des sinus selon la méthode de Proetz ou pour être aérosolisée, ou bien encore sous la forme de solution pour l'utilisation en flacon compte-gouttes.

L'administration des compositions pharmaceutiques selon l'invention s'effectue généralement 2 à 3 fois par jour, mais elle peut varier selon la gravité de l'affection à traiter et du type de traitement : curatif ou préventif.

On donnera ci-après à titre d'exemples particulièrement préférés aux fins de l'invention les deux compositions suivantes :

| Composition A | | Composition B | |
|---|---|---|---|
| (mg/l) | | (mg/l) | |
| ClNa | 8 | ClNa | 8 |
| ClK | 240 | ClK | 300 |
| Cl₂Mg (6H₂O) | 550 | Cl₂Mg (6H₂O) | 20 |
| Cl₂Ca (6H₂O) | 1 | Cl₂Ca (6H₂O) | 0,85 |
| Mannitol | 800 | Mannitol | 400 |
| D-Glucose | 7 250 | D-Glucose | 6 250 |
| Chlorhydrate de | | Chlorhydrate de | |
| L-Arginine | 2343 | L-Arginine | 2343 |
| Gluconate de cuivre * | 7,25 | Chlorure de zinc ** | 7,20 |
| Eau bidistillée Q.S.P | 1 000 cc | Eau bidistillée Q.S.P | 1 000 cc |

| | | | |
|---|---|---|---|
| * quantité correspondante en cuivre métal | | | |
| ** quantité correspondante en zinc métal | | | |

La pratique de l'ORL permet de vérifier chaque jour l'efficacité de ces compositions sur de nombreuses affections des voies respiratoires supérieures,

Deux particularités physioanatomiques des muqueuses nasales, l'une propre aux fosses nasales, l'autre caractéristique des muqueuses respiratoires permettent de visualiser :
- l'action dépolarisante de la composition A sur des muscles lisses unitaires dérégulés par une hyperpolarisation,
- l'action polarisante de la composition B sur des muscles lisses unitaires dérégulés par une hyperdépolarisation.

En effet la présence d'un réseau vasculaire avec du tissu érectile amplifie considérablement toute manifestation vasomotrice, et l'absence de tissu lâche interposé entre la muqueuse respiratoire et le squelette sous-jacent, fait que toutes les manifestations vasomotrices se font aux dépens de la lumière nasale et obstruent ou au contraire, perméabilisent fosse nasale et méats.

Ainsi la simple rhinoscopie antérieure permet :
- d'observer toutes les modifications volumiques et sécrétoires de la muqueuse et cela d'autant plus facilement que ces modifications s'effectuent en quelques minutes voire pour certaines en quelques dizaines de seconde,
- d'interpréter les mécanismes mis en jeu, sachant que la dépolarisation contracte le muscle lisse et vide la cellule sécrétoire de ses sécrétions en contractant la myofibrille ; et que la polarisation a les effets inverses.

Deux formes cliniques d'obstruction nasale, par troubles vaso-moteurs d'étiopathogénies différentes, peuvent servir d'exemple :
- l'une d'origine exogène est initiée par la dessiccation de la muqueuse : la chaleur alors développée par le frottement des contaminants aéroportés sur l'épithélium déclenche, par réflexe vagal, une vaso-dilatation artérioveineuse et une hypersécrétion séromuqueuse transitoire par étirement passif des myofibrilles des glandes sous muqueuses. Aussi l'examen pratiqué quelques heures plus tard montre-t-il des fosses nasales et des méats obstrués par une muqueuse congestive et souvent recouverte de croutelles de mucus desséché.
- l'autre exemple, d'origine endogène peut être initié par l'intervention intempestive de divers médiateurs à effet dépolarisant sur le sphincter capillaire veinulaire. La contraction tonique de ce sphincter entraîne un oedème mécanique obstruant fosse nasale et méat avec pâleur de la muqueuse et arrêt des sécrétions.

Avantageusement, on administrera séquentiellement les compositions A et B, par exemple par pulvérisation. Ainsi, on peut administrer tout d'abord la composition A puis, environ 1 à 2 minutes après, la composition B, ou inversement.

Par exemple, dans le cas de l'obstruction nasale d'origine exogène, la pulvérisation de la composition A entraîne rapidement (quelques dizaines de secondes) la rétraction de la muqueuse et la perméabilisation de la fosse nasale et des méats et une importante sécrétion séro-muqueuse. La pulvérisation de la composition B, pratiquée 1 à 2 minutes après, en régularisant la sécrétion séro-muqueuse, restitue à la muqueuse son aspect luisant et apporte la sensation de respirer "plus profondément et plus frais".

Dans le cas de l'obstruction nasale d'origine endogène, la pulvérisation de la composition B (pratiquée 1 à 2 minutes environ après celle de la composition A) entraîne en 10 à 20 minutes la perméabilisation de la fosse nasale et des méats et restitue à la muqueuse un volume et un aspect normal (coloration et brillant). La décontraction du sphincter veinulaire obtenue par sa polarisation a levé la pression capillaire génératrice de l'oedème mécanique.

La présence de gluconate de cuivre dans la composition A et de chlorure de Zn dans la composition B a montré une synergie d'action de ces solutions dans les états inflammatoires aigus et surtout subaïgus.

La preuve de cette synergie d'action a été apportée par une étude portant sur 2 séries de 10 malades adultes atteints de virose des voies respiratoires supérieures avec otite bilatérale et manoeuvres d'équipression tubaire négatives.

### Autres critères d'inclusion :

- Subjectifs : Otalgies bilatérales plus ou moins vives
   Hypoacousie bilatérale confirmée par le test de la voix chuchotée.
- Objectifs : Tympans congestifs et rétractés avec ou sans exsudat de la caisse.

### Critères d'exclusion :

- Obstruction nasale unilatérale ou bilatérale liée à une importante déviation de cloison, ou une dégénérescence polypoïde de la muqueuse ou une autre pathologie associée sous-jacente.
- Otite suraiguë, otite phlycténulaire, otite suppurée.

L'expérimentation va consister à comparer les résultats obtenus sur l'otite homolatérale, après avoir pulvérisé :
Dans la 1ère série : composition A + composition B dans la fosse nasale I et Cu + Zn dans la fosse nasale II
Dans la 2ème série : composition A + composition B dans la fosse nasale I et A₁ + B₁ dans la fosse nasale II (A₁ et B₁ correspondent aux compositions A et B sans gluconate de cuivre et, respectivement, sans chlorure de zinc).

### Remarques :

- La fosse nasale I est celle qui correspond à l'oreille paraissant la plus atteinte cliniquement.
- Est considéré comme résultat positif, la perméabilisation de la trompe d'Eustache correspondant à la fosse nasale pulvérisée, obtenue dans un délai d'une heure, avec manoeuvres d'équipression tubaire positives, et disparition ou amélioration des signes fonctionnels et objectifs.

### Résultats :

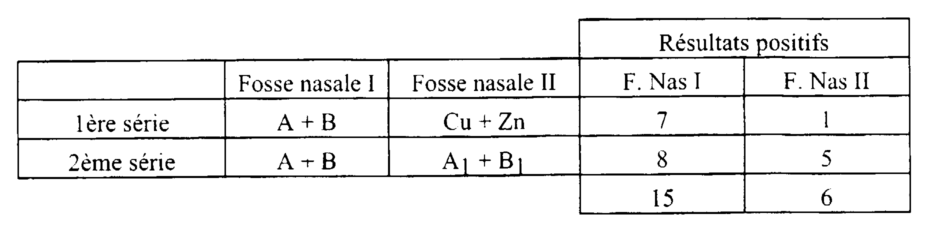

### CONCLUSION : L'action globale de l'association de 2 principes

- d'une part 2 solutés à pulvériser successivement dans les fosses nasales pour le traitement des affections récidivantes des voies respiratoires (A₁ + B₁),
- d'autre part un soluté de cuivre et de zinc utilisé en oligothérapie dans les états infectieux (Cu + Zn),
   est supérieure à l'addition de leurs 2 effets lorsqu'ils sont séparés, pour le traitement des états inflammatoires aigus et surtout subaigus des voies respiratoires avec complication sinusienne ou (et) auriculaire ou (et) pharyngée.

## Revendications

1. Composition pharmaceutique apte à réguler la polarisation des muscles lisses unitaires, caractérisée en ce qu'elle est constituée d'une solution aqueuse contenant des sels de magnésium, de potassium et de sodium, et éventuellement de calcium, au moins un sucre et au moins un acide aminé en combinaison avec un sel de cuivre ou un sel de zinc, la quantité de sels étant comprise entre 10 et 2000 mg/l, la quantité d'acide aminé étant comprise entre 1 et 20 g/l et celle du sucre entre 200 et 10 000 mg/l, la quantité de cuivre métal étant comprise entre 5 et 100 mg/l et celle de zinc métal entre 5 et 120 mg/l.

2. Composition selon la revendication 1, caractérisée en ce que le rapport pondéral K/Mg est compris entre 0,40 et 0,85.

3. Composition selon la revendication 1, caractérisée en ce que le rapport pondéral K/Mg est compris entre 14 et 16.

4. Composition selon la revendication 3, caractérisée en ce que les sels de magnésium, potassium, sodium et éventuellement calcium sont des chlorures et en ce que le rapport pondéral K/Na est compris entre 30 et 40.

5. Trousse contenant au moins deux compositions pharmaceutiques selon la revendication 1, pour la régulation de la polarisation des muscles lisses unitaires par administration simultanée, successive ou séparée desdites compositions.

6. Trousse selon la revendication 5, contenant deux compositions pharmaceutiques, l'une ayant un effet polarisant sur les muscles lisses unitaires, l'autre ayant un effet dépolarisant sur les muscles lisses unitaires.

7. Trousse selon la revendication 6, caractérisée en ce qu'une des compositions pharmaceutiques a un rapport pondéral K/Mg compris entre 0,4 et 0,85, et l'autre un rapport pondéral K/Mg compris entre 14 et 16.

8. Trousse selon la revendication 7, caractérisée en ce que la composition pharmaceutique ayant un rapport pondéral K/Mg compris entre 14 et 16 possède de plus un rapport pondéral K/Na compris entre 30 et 40 et en ce que les sels de magnésium, potassium, sodium et éventuellement calcium de ladite composition sont des chlorures.

## Patentansprüche

1. Pharmazeutische Zusammensetzung zur Regulierung der Polarisation der unitären glatten Muskulatur, dadurch gekennzeichnet, daß sie aus einer wässrigen Lösung besteht, die Magnesium-, Kalium-, Natrium- und ggf. Calciumsalze, mindestens einen Zucker und mindestens eine Aminosäure in Kombination mit einem Kupfersalz oder Zinksalz enthält, wobei die Salzmenge zwischen 10 und 2000 mg/l, die Aminosäuremenge zwischen 1 und 20 g/l und die Zuckermenge zwischen 200 und 10 000 mg/l beträgt und die Kupfermetallmenge zwischen 5 und 100 mg/l und die Zinkmetallmenge zwischen 5 und 120 mg/l beträgt.

2. Zusammensetzung nach Anspruch 1, dadurch gekennzeichnet, daß das Gewichtsverhältnis K/Mg zwischen 0,40 und 0,85 beträgt.

3. Zusammensetzung nach Anspruch 1, dadurch gekennzeichnet, daß das Gewichtsverhältnis K/Mg zwischen 14 und 16 beträgt.

4. Zusammensetzung nach Anspruch 3, dadurch gekennzeichnet, daß die Magnesium-, Kalium-, Natrium- und ggf. Calciumsalze Chloride sind und daß das Gewichtsverhältnis K/Na zwischen 30 und 40 beträgt.

5. Kit, enthaltend mindestens zwei pharmazeutische Zusammensetzungen nach Anspruch 1 zur Regulierung der Polarisation der unitären glatten Muskulatur durch gleichzeitige, aufeinanderfolgende oder getrennte Verabreichung dieser Zusammensetzungen.

6. Kit nach Anspruch 5, enthaltend zwei pharmazeutische Zusammensetzungen, deren eine eine polarisierende Wirkung auf die unitäre glatte Muskulatur hat und deren andere eine depolarisierende Wirkung auf die unitäre glatte Muskulatur hat.

7. Kit nach Anspruch 6, dadurch gekennzeichnet, daß eine der pharmazeutischen Zusammensetzungen ein Gewichtsverhältnis K/Mg zwischen 0,4 und 0,85 und die andere ein Gewichtsverhältnis K/Mg zwischen 14 und 16 hat.

8. Kit nach Anspruch 7, dadurch gekennzeichnet, daß die pharmazeutische Zusammensetzung mit einem Gewichtsverhältnis K/Mg zwischen 14 und 16 außerdem ein Gewichtsverhältnis K/Na zwischen 30 und 40 hat und daß die Magnesium-, Kalium-, Natrium- und ggf. Calciumsalze dieser Zusammensetzung Chloride sind.

## Claims

1. Pharmaceutical composition capable of regulating unitary smooth muscle polarization, characterized in that it consists of an aqueous solution containing magnesium, potassium, sodium and optionally calcium salts, at least one sugar and at least one amino acid in combination with a copper salt or a zinc salt, the amount of salts being between 10 and 2000 mg/l, the amount of amino acid being between 1 and 20 g/l, the amount of sugar being between 200 and 10,000 mg/l, the amount of copper metal being between 5 and 100 mg/l and the amount of zinc metal being between 5 and 120 mg/l.

2. Composition according to claim 1, characterized in that the weight ratio K/Mg is between 0.40 and 0.85.

3. Composition according to claim 1, characterized in that the weight ratio K/Mg is between 14 and 16.

4. Composition according to claim 3, characterized in that the magnesium potassium, sodium and optionally calcium salts are chlorides and in that the weight ration K/Na is between 30 and 40.

5. Kit containing at least two pharmaceutical compositions according to claim 1 for regulating unitary smooth muscle polarization by the simultaneous, successive or separate administration of said compositions.

6. Kit according to claim 5 containing two pharmaceutical compositions, one having a polarizing effect on the unitary smooth muscles and the other having a depolarizing effect on the unitary smooth muscles.

7. Kit according to claim 6, characterized in that one of the pharmaceutical composition has a weight ratio K/Mg of between 0.4 and 0.85 and the other has a weight ratio K/Mg of between 14 and 16.

8. Kit according to claim 7, characterized in that the pharmaceutical composition with a weigh ration K/Mg of between 14 and 16 also has a further weight ratio K/Na of between 30 and 40, and in that the magnesium, potassium, sodium and optionally calcium salts of said composition are chlorides.
